(19) Europäisches Patentamt
European Patent Office
Office européen des brevets

(11) Veröffentlichungsnummer: **0 167 071**
**A2**

# (12) EUROPÄISCHE PATENTANMELDUNG

(21) Anmeldenummer: 85107673.7

(22) Anmeldetag: 21.06.85

(51) Int. Cl.[4]: C 07 H 23/00
A 61 K 31/70

(30) Priorität: 30.06.84 DE 3424217

(43) Veröffentlichungstag der Anmeldung: 08.01.86 Patentblatt 86/2

(84) Benannte Vertragsstaaten: AT BE CH DE FR GB IT LI LU NL SE

(71) Anmelder: BEHRINGWERKE Aktiengesellschaft
Postfach 1140
D-3550 Marburg 1(DE)

(72) Erfinder: Kolar, Cenek, Dr.
Deutschhausstrasse 20
D-3550 Marburg 1(DE)

(72) Erfinder: Kraemer, Hans-Peter, Dr.
Birkenweg 16
D-3550 Marburg(DE)

(72) Erfinder: Dehmel, Konrad
Blumengarten 11
D-3550 Marburg-Michelbach(DE)

(74) Vertreter: Meyer-Dulheuer, Karl-Hermann, Dr. et al,
HOECHST Aktiengesellschaft Zentrale Patentabteilung
Postfach 80 03 20
D-6230 Frankfurt/Main 80(DE)

## (54) Cis-Platin(II)-Komplexe mit Diaminozucker-Derivaten als Liganden, Verfahren zu ihrer Herstellung und diese enthaltendes Arzneimittel.

(57) Es werden Verbindungen der allgemeinen Formel I beschrieben,

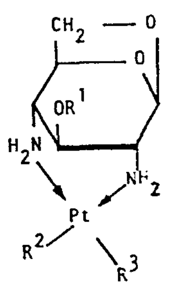

I

worin

$R^1$ ein Wasserstoffatom, eine Alkylgruppe mit 1 bis 16 Kohlenstoffatomen oder eine in der Kohlenhydratchemie übliche Schutzgruppe, besonders eine Benzyl-, Tetrahydropyranyl-, Acetyl-, Halogenacetyl-, Benzoyl-, Methansulfonyl- oder para-Toluolsulfonyl-Gruppe,

$R^2$ und $R^3$ gleich sind und Chlorid, Bromid oder Jodid oder Hydroxy, Nitrat, Acetat, Trifluoracetat, Trifluormethansulfonat, Toluolsulfonat oder Perchlorat oder

$R^2$ Sulfat oder Carbonat und $R^3$ $H_2O$ oder

$R^2$ und $R^3$ zusammen das Dianion einer organischen Säure, besonders der Oxal-, Malon-, Hydroxymalon-, Bernstein-, Malein-, Aconit-, 3,6,9-Trioxaundecandi-, 1,1- oder 1,2-Cyclobutandicarbon-, 3- oder 4-Carboxyphthal- oder 3,4-Dicarboxyphthalsäure oder

$R^2$ und $R^3$ zusammen eine wiederkehrende anionische Einheit einer polymeren Verbindung, besonders Dextran-, Chondroitin- oder Galactansulfat, Polyglutaminsäure oder Polyitaconsäure darstellen, sowie ein Verfahren zu ihrer Herstellung und ein diese Verbindungen enthaltendes Arzneimittel.

EP 0 167 071 A2

0167071

EHRINGWERKE AKTIENGESELLSCHAFT

84/B 013 - Ma 452
Dr. Ha/Sd.

Cis-Platin(II)-Komplexe mit Diaminozucker-Derivaten als Liganden, Verfahren zu ihrer Herstellung und diese enthaltendes Arzneimittel

---

Die Erfindung betrifft Verbindungen der allgemeinen Formel I

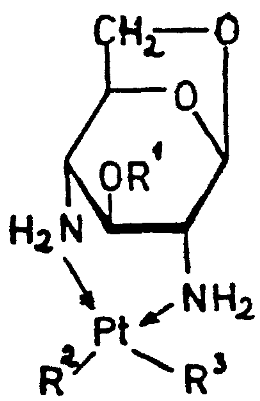

I

worin

$R^1$ ein Wasserstoffatom, eine Alkylgruppe mit 1 bis 16 Kohlenstoffatomen oder eine in der Kohlenhydratchemie übliche Schutzgruppe, besonders eine Benzyl-, Tetrahydropyranyl-, Acetyl-, Halogenacetyl-, Benzoyl-, Methansulfonyl- oder para-Toluolsulfonyl-Gruppe,

$R^2$ und $R^3$ gleich sind und Chlorid, Bromid oder Jodid oder Hydroxy, Nitrat, Acetat, Trifluoracetat, Trifluormethansulfonat, Toluolsulfonat oder Perchlorat oder

$R^2$ Sulfat oder Carbonat und $R^3$ $H_2O$ oder

$R^2$ und $R^3$ zusammen das Dianion einer organischen Säure, besonders der Oxal-, Malon-, Hydroxymalon-, Bernstein-, Malein-, Aconit-, 3,6,9-Trioxaundecandi-, 1,1- oder 1,2-Cyclobutandicarbon-, 3- oder 4-Carboxyphthal- oder 3,4-Dicarboxyphthalsäure oder

$R^2$ und $R^3$ zusammen eine wiederkehrende anionische Einheit einer polymeren Verbindung, besonders Dextran-, Chondroitin- oder Galactansulfat, Polyglutaminsäure oder Polyitaconsäure darstellen, ein Verfahren zu ihrer Herstellung und ein diese Verbindungen enthaltendes Arzneimittel.

Es ist bekannt, daß cis-Platin-Komplexe der allgemeinen Formel cis-$L_2PtX_2$, wobei L ein neutraler Ligand, wie $NH_3$ oder organisches Amin und X ein anionischer Ligand, wie Chlorid oder Sulfat ist, Antitumorwirkung besitzen (Cisplatin - Current Status and New Developments; Edts.: A.W. Prestayko, S.T. Crooke, S.K. Carter, Academic Press, 1980). In DE 31 08 842 Al sind cis-Platin(II)-Komplexe beschrieben, deren Liganden zwei gleiche Aminozucker sind. Zum Beispiel aus L.M. Hall, J.Clin.Hemat.Oncol. 7/1, 231, 1977, ist es bekannt, daß in dem cis-Platin-Komplex die Amino-Liganden die zytostatische Wirksamkeit und die anionischen Liganden die Toxizität bestimmen.

Cis-$(NH_3)_2PtCl_2$ ist als Arzneimittel eingeführt.

Die vorliegende Erfindung hatte sich zur Aufgabe gestellt, cis-Platin-Komplexe herzustellen, in denen ein Diaminozucker als zweizähniger Ligand fungiert und eine therapeutisch vertretbare anionische Verbindung als Ligand die pharmakologischen und toxikologischen Eigenschaften günstig beeinflußt.

Gelöst wurde diese Aufgabe durch die Herstellung von Verbindungen mit der voranstehenden Formel I und den zu dieser Formel angegebenen Definitionen.

Es wurde überraschenderweise gefunden, daß die 2,4-Diaminozucker-cis-Platin-Komplexe der allgemeinen Formel I zytostatisch wirksam sind und eine wesentlich niedrigere Toxizität als die Stammverbindung cis-$(NH_3)PtCl_2$ aufweisen.

Bevorzugt werden im Rahmen der Erfindung Verbindungen der allgemeinen Formel I, worin $R^1$ ein Wasserstoffatom, eine Alkylgruppe mit 1-10 Kohlenstoffatomen oder eine Benzyl-, Tetrahydropyranyl-, Acetyl- oder Methansulfonylgruppe,
$R^2$ und $R^3$ gleich sind und Chlorid, Jodid, Nitrat, Hydroxy, Acetat, Trifluoracetat, Trifluorsulfonat, Toluolsulfonat oder Perchlorat oder
$R^2$ Sulfat oder Carbonat und $R^3$ $H_2O$ oder
$R^2$ und $R^2$ zusammen das Dianion einer organischen Säure, besonders der Oxal-, Malon-, Hydroxymalon-, Bernstein-, Malein-, Aconit-, 3,6,9-Trioxaundecandi-, 1,1- oder 1,2-Cyclobutandicarbon-, 3- oder 4-Carboxyphthal- oder 3,4-Dicarboxyphthalsäure oder
$R^2$ und $R^3$ zusammen eine wiederkehrende anionische Einheit einer polymeren Verbindung, besonders Dextransulfat, Chondroitinsulfat, Galactansulfat, Polyglutaminsäure oder Polyitaconsäure darstellen.

Das erfindungsgemäße Verfahren zur Herstellung der Verbindungen der Formel I ist dadurch gekennzeichnet, daß man

a) eine Verbindung der allgemeinen Formel II

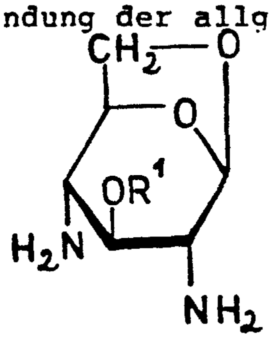

II

in der $R^1$ ein Wasserstoffatom oder eine Alkylgruppe mit 1 bis 16 Kohlenstoffatomen oder eine in der Kohlenhydratchemie übliche Schutzgruppe, besonders eine Benzyl-, Tetrahydropyranyl-, Acetyl-, Halogenacetyl-, Benzoyl-, Methansulfonyl- oder para-Toluolsulfonyl-Gruppe ist, mit

$K_2PtX_4$, worin X ein Chlorid- oder Jodid-Ion darstellt, in Gegenwart eines Lösungsmittels wie Wasser, DMF oder DMSO oder deren Mischungen mit THF, Dioxan, Methanol oder Ethylenglycoldimethylether bei einer Temperatur von 0°C bis 80°C, bevorzugt 10°C bis 40°C, in an sich bekannter Weise zu einem Reaktionsprodukt der allgemeinen Formel I umsetzt, in der $R^1$ die oben angegebene Bedeutung besitzt und $R^2$ und $R^3$ Chlorid oder Jodid bedeuten,

b) das Produkt von Stufe a) in an sich bekannter Weise mit einer anorganischen oder organischen Silber-I-Verbindung wie Silber-oxid, -nitrat, -sulfat, -carbonat, -perchlorat, -acetat, -trifluoracetat, -trifluormethansulfonat oder -para-toluolsulfonat in Gegenwart von Wasser oder einem organischen Lösungsmittel wie DMF, DMSO, THF, Dioxan oder Methanol oder deren wäßrigen Mischungen zu einer Verbindung der allgemeinen Formel I umsetzt, worin $R^2$ und $R^3$ das Anion der eingesetzten Silberverbindung darstellen,

c) in dem Produkt von Stufe b) in an sich bekannter Weise die anionischen Liganden $R^2$ und $R^3$, bevorzugt Nitrat oder Perchlorat, gegen eine anionische Verbindung, die sich von einem Alkali-Salz einer Carbon- oder Sulfonsäure ableitet, austauscht. Solche anionischen Verbindungen sind Dicarbonsäuren wie Oxal-, Malon-, Hydroxymalon-, Bernstein-, Maleinsäure, Phthalsäure, 1,1- oder 1,2-Cyclobutandicarbonsäure, 3,6,9-Trioxaundecandisäure, Tri- oder Oligocarbonsäuren wie Aconitsäure, 3- oder 4-Carboxyphthalsäure, 3,4-Dicarboxyphthalsäure, polymere Verbindungen wie Polyglutaminsäure, Polyitaconsäure, Dextransulfat, Chondroitinsulfat oder Galactansulfat.

Gegenstand der Erfindung sind auch Arzneimittel, die eine oder mehrere der Verbindungen der Formel I als Wirkstoff enthalten. Neben den üblichen pharmazeutischen

Konfektionierungs- und/oder Verdünnungsmitteln können diese Arzneimittel neben den Verbindungen der Formel I zur Unterstützung der Therapie gegebenenfalls auch noch weitere Wirkstoffe enthalten, sofern diese mit den erfindungsgemäßen Verbindungen der Formel I zusammen keine unerwünschten Nebenwirkungen zeigen. Die Dosierungs- und Anwendungsweise entspricht im wesentlichen der für cis-$(NH_3)_2PtCl_2$ bekannten, wobei aber aufgrund der größeren therapeutischen Breite (wesentlich geringere Toxizität) der erfindungsgemäßen Verbindungen auch höhere Dosierungen und/oder eine häufigere Verabreichung in Frage kommen.

Ermittlung der zytotoxischen Aktivität

Die Bestimmung der zytostatischen Wirksamkeit der hier beschriebenen Verbindungen erfolgte an L1210 Leukämiezellen der Maus. Im einzelnen wurden folgende Testsysteme verwendet:

a) Proliferationsassay

Bei dieser Methode wird in vitro nach Inkubation der Zellen mit unterschiedlichen Konzentrationen der Testsubstanz ermittelt, inwieweit die Zellen radioaktiv markierte DNA-Vorläufer (z.B. C14 markiertes Thymidin) einbauen können. Unbehandelte L1210 Zellen werden den gleichen Testbedingungen unterworfen und dienen als Kontrolle. Im folgenden sei die Methode kurz beschrieben:

L1210 Zellen in expotentieller Wachstumsphase ($5x10^3$/ ml in RPMl 1640) werden in einer Mikrotitrationsplatte 72 Stunden mit unterschiedlichen Konzentrationen der Testsubstanz inkubiert (37°C, 5 % $CO_2$, 95 % relative

Luftfeuchte). Kontrollen bestehen aus Zellen, die lediglich mit frischem Medium inkubiert werden. Alle Bestimmungen werden als 4-fach Bestimmungen durchgeführt. Nach 65 Stunden werden 50 µl C14 Thymidin (1,5 µc/ml) zugegeben, um die DNA der Zelle radioaktiv zu markieren. Nach 7 Stunden Inkubation werden die Zellen abgesaugt, die DNA mit 5 %iger Trichloressigsäure gefällt und nacheinander mit Wasser oder Methanol gewaschen.
Nach Trocknung bei 50°C wird die in die DNA eingebaute Radioaktivität nach Zugabe von 5 ml Szintilationsflüssigkeit ermittelt.
Die Ergebnisse werden angegeben als Verhältnis des Szintilationsindex nach Inkubation mit der Testsubstanz in Prozent der unbehandelten Kontrolle. Aus den so erhaltenen Meßwerten wird die Dosiswirkungs kurve ermittelt und grafisch die $IC_{50}$, d.h. die Konzentration, die unter Testbedingungen den Einbau von radioaktivem Thymidin um 50% gegenüber der Kontrolle erniedrigt, ermittelt. Die $IC_{50}$ Werte der hier beschriebenen Verbindungen im Vergleich zu Cisplatin (DDP) wurden in der Tabelle 1 zusammengefaßt.

b) Koloniebildung von L1210 Leukämiezellen in soft agar

Diese Methode dient zum Nachweis eines Einflusses der Testsubstanzen auf das Wachstumsverhalten der Zellen über mehrere Generationen (bei einer Zellzykluszeit von 10-12 Stunden werden in der Testzeit von 7 Tagen etwa 14 aufeinanderfolgende Generationen beobachtet). Zytostatisch wirksame Substanzen bewirken in diesem Test eine Reduktion der zu beobachtenden Koloniezahl gegenüber einer unbehandelten Kontrolle. Im einzelnen wird der Test wie folgt durchgeführt:

500 Leukämiezellen pro Platte werden mit unterschiedlichen Konzentrationen von Testsubstanz 1 Stunde bei 37°C inkubiert. Anschließend werden die Zellen zweimal mit McCoy5A-Medium gewaschen und schließlich in Petrischalen nach Zusatz von 0.3 % Agar ausgegossen. Kontrollen werden lediglich mit frischen Medium inkubiert. Anstelle der ein-stündigen Inkubation wird in manchen Fällen unterschiedliche Konzentrationen und Testsubstanz der oberen Agarschicht zugemischt, um so eine kontinuierliche Exposition der Zellen über die gesamte Inkubationszeit zu erreichen. Nach Erstarren des Agars werden die Platten im Brutschrank 7 Tage bei 37°C inkubiert (5 % $CO_2$, 95 % relative Luftfeuchtigkeit). Anschließend wird die Anzahl der entstandenen Kolonien mit einem Durchmesser von mehr als 60 µ gezählt. Die Ergebnisse werden angegeben als Koloniezahl in behandelten Agarplatten in Prozent der unbehandelten Kontrolle. Aus der so erhaltenen Dosiswirkungskurve wird die $IC_{50}$ als Maß für die Wirksamkeit der Substanz ermittelt. Die Ergebnisse für die hier beschriebenen Verbindungen im Vergleich zu Cisplatin sind in der Tabelle 1 zusammengefaßt.

c) Ermittlung der akuten Toxizität

Zur Ermittlung der akuten Toxizität werden NMRI-Mäusen am Tag 0 unterschiedliche Dosen der Testsubstanz, gelöst in 0.5 ml physiologischer Kochsalzlösung, intravenös injiziert. Kontrollgruppen erhalten lediglich 0,5 ml physiologischer Kochsalzlösung. Pro Konzentration der Testsubstanz werden 5 Mäuse verwendet. Am Tag 14 wird die Zahl der überlebenden Mäuse ermittelt und daraus nach der Litchfield Wilcoxon Methode die LD5, LD50 und LD95 ermittelt. Die Toxizität der hier beschriebenen Verbindungen im Vergleich zu Cisplatin ist in Tabelle 1 zusammengefaßt.

Tabelle 1

Ermittlung der zytotoxischen Aktivität

| Verbindung | Testsystem | | |
|---|---|---|---|
| | a | b | c |
| 15 | 0,22 | 0,33 | 79,8 |
| 16 | 2,0 | 0,7 | - |
| 17 | 3,2 | 1,2 | - |
| 18 | 1,2 | 3,0 | - |
| 19 | 2,5 | 2,8 | - |
| 20 | 2,8 | 2,7 | - |
| 21 | 0,4 | 0,72 | - |
| 23 | 0,8 | 0,25 | - |
| 24 | 0,29 | 0,1 | - |
| 26 | 1,8 | 0,45 | - |
| 28 | 0,36 | 0,55 | 17 |
| 29 | 0,32 | 0,2 | 22 |
| 30 | 0,38 | 0,57 | 187 |
| 34 | 0,83 | 0,62 | - |
| 35 | 0,24 | 0,15 | 416 |
| 37 | 0,7 | 0,27 | >200 |
| 38 | 0,32 | 0,14 | >200 |
| 39 | 0,34 | 0,13 | >200 |
| 40 | 0,47 | 0,29 | 36,5 |

Die folgenden Beispiele erläutern die Erfindung näher, ohne sie darauf zu beschränken.

**Beispiel 1** (Herstellung von 2,4-Diazido-Verbindungen)

1,6-Anhydro-2,4-di-azido-2,4-di-desoxy-ß-D-glucopyranose (Verbindung 1)

Die Verbindung 1 wurde nach der Vorschrift von H. Koebernick, Dissertation, Hamburg 1975, hergestellt.

1,6-Anhydro-2,4-di-azido-2,4-di-desoxy-3-O-methyl-ß-D-glucopyranose (Verbindung 2)

5 g (23,5 mMol) Verbindung 1 wurden in 50 ml trockenem Dioxan gelöst. Unter Feuchtigkeitsausschluß wurden 3,96 g Kalium tert.-butylat und 4,0 g Methyljodid gelöst in 30 ml Dioxan zugegeben. Nach 20 Minuten Rühren bei Raumtemperatur war die Reaktion beendet (Dünnschichtchromatographie-Kontrolle in Chloroform/Methanol 10:1). Die Reaktionsmischung wurde i.Vak. eingeengt. Der in Chloroform aufgenommene Rückstand wurde zweimal mit Wasser gewaschen. Die organische Phase wurde mit Natriumsulfat getrocknet und i.Vak. eingeengt. Das Rohprodukt wurde noch säulenchromatographisch (über 250 g Kieselgel, Elutionsmittel Chloroform/Aceton 4:1) gereinigt.

| | |
|---|---|
| Ausbeute: | 5 g (Sirup) |
| IR ($cm^{-1}$): | 3000-2860, 2100, 1120 |

| | | | |
|---|---|---|---|
| berechnet (%): | C 37,17 | H 4,45 | N 37,15 |
| gefunden (%): | C 37,20 | H 4,46 | N 37,03 |

Analog der Verbindung 2 wurde die folgende Verbindung synthetisiert:
1,6-Anhydro-2,3-di-azido-3-O-decyl-2,4-di-desoxy-ß-D-glucopyranose (Verbindung 3)

IR ($cm^{-1}$): 3000-2860, 2100, 1120

| | | | |
|---|---|---|---|
| berechnet (%): | C 54,52 | H 8,00 | N 23,84 |
| gefunden (%): | C 54,61 | H 8,03 | N 23,56 |

1,6-Anhydro-2,4-di-azido-3-0-benzyl-2,4-di-desoxy-ß-D-glucopyranose (Verbindung 4)

5 g (23,5 mMol) Verbindung 1 wurden in 100 ml trockenem DMF gelöst. Nach der Zugabe von 7,2 g BaO und 9,3 g $Ba(OH)_2$ x 7 $H_2O$ wurden der gerührten Suspension 4 g Benzylbromid gelöst in 30 ml DMF zugetropft. Nach 90 min Rühren unter Feuchtigkeitsausschluß war die Reaktion beendet. Die Supension wurde abfiltriert und das Filtrat wurde i.Vak. eingeengt. Das einheitliche Rohprodukt wurde noch säulenchromatographisch gereinigt (Elutionsmittel: Chloroform/Esssigester 3:1).

Ausbeute: 5,5 g (77,5 %)
IR ($cm^{-1}$): 3100-2850, 2100, 1500, 1460

| | | | |
|---|---|---|---|
| berechnet (%): | C 51,65 | H 4,67 | N 27,80 |
| gefunden (%): | C 51,71 | H 4,64 | N 27,73 |

1,6-Anhydro-2,4-di-azido-2,4-di-desoxy-3-0-tetrahydropyranyl-ß-D-glucopyranose (Verbindung 5)

5 g (23,5 mMol) Verbindung 1 wurden in 50 ml trockenem Dioxan gelöst und mit 2,7 g 4,5-Dihydropyran und 50 mg p-Toluolsulfonsäure versetzt. Die Lösung wurde 6 Stunden bei Raumtemperatur und anschließend noch 1 Stunde bei 50°C gerührt (Dünnschichtchromatogramm: Chloroform/Essigester 6:1). Der Reaktionsmischung wurde Chloroform und Wasser zugegeben. Die organische Phase wurde noch zweimal mit Wasser gewaschen und anschließend mit Natriumsulfat getrocknet. Nach Einengen der organischen Phase i.Vak. wurde der resultierende Sirup säulenchromatographisch gereinigt (Elutionsmittel: Chloroform/Essigester 9:1).

Ausbeute: 5,7 g (82 %)
IR ($cm^{-1}$): 3000-2900, 2100

| | | | |
|---|---|---|---|
| berechnet (%): | C 44,59 | H 5,44 | N 28,36 |
| gefunden (%): | C 44,32 | H 5,41 | N 28,18 |

1,6-Anhydro-2,4-di-azido-2,4-di-desoxy-3-O-methansulfonyl-ß-D-glucopyranose (Verbindung 6)

5 g (23,5 mMol) Verbindung 1 wurden in 50 ml trockenem Pyridin gelöst. Unter Kühlen wurden der gerührten Lösung 2,9 g Methansulfonsäurechlorid zugetropft. Danach wurde die Reaktionsmischung noch 10 Stunden bei Raumtemperatur gerührt (Dünnschichtchromatogramm: Chloroform/Methanol 10:1). Nach Einengen des Reaktionsgemisches i.Vak. wurde der resultierende Sirup in Chloroform gelöst und zweimal mit Wasser ausgeschüttelt. Die organische Phase wurde über Natriumsulfat gerocknet und i.Vak. eingeengt. Das Produkt wurde noch säulenchromatographisch gereinigt (Elutionsmittel: Chloroform/Aceton 4:1).

Ausbeute: 5,8 g (86 %)
IR ($cm^{-1}$): 3040-2900, 2100, 1370, 1250, 1180, 1135

| | | | | |
|---|---|---|---|---|
| berechnet (%): | C 28,96 | H 3,47 | N 28,95 | S 11,04 |
| gefunden (%): | C 28,74 | H 3,48 | N 28,91 | S 10,91 |

3-O-Acetyl-1,6-anhydro-2,4-di-azido-2,4-di-desoxy-ß-D-glucopyranose (Verbindung 7)

5 g (23,5 mMol) Verbindung 1 wurden mit Acetanhydrid in

Gegenwart von Pyridin umgesetzt. Der Reaktionsansatz wurde nach den in der Kohlenhydratchemie üblichen Methoden aufgearbeitet.

Ausbeute: 5,45 g (91 %)
IR ($cm^{-1}$): 3000-2900, 2100, 1740

| | | | |
|---|---|---|---|
| berechnet (%): | C 37,80 | H 3,96 | N 33,06 |
| gefunden (%): | C 37,83 | H 3,97 | N 33,02 |

Beispiel 2 (Herstellung von 2,4-Diamino-Verbindungen)

2,4-Di-amino-1,6-anhydro-2,4-di-desoxy-ß-D-glucopyranose (Verbindung 8)

1,0 g (6,2 mMol) Verbindung 1 wurden in 20 ml eines Lösungsmittelgemisches bestehend aus Essigester/Methanol 2:1 in Gegenwart von 1 g Palladium-Kohle (10 %) hydriert. Das Produkt wurde dünnschichtchromatographisch verfolgt (Laufmittel: Chloroform/Methanol 10:1, Entwicklungsreagenz: Ninhydrin). Nach 1 Stunde wurde der Reaktionsansatz sorgfältig abfiltriert und das Filtrat wurde i.Vak. zum Sirup eingeengt. Das resultierende Produkt wurde zuerst IR-spektroskopisch charakterisiert (Abwesenheit der Azidobande bei 2100 $cm^{-1}$) und dann zur Herstellung der folgenden cis-Platin-Derivate unmittelbar verwendet.
IR ($cm^{-1}$): 3400-3250, 3000, 2900, 1600

Die Verbindungen 2 bis 7 wurden nach der Vorschrift zur Herstellung der Verbindung 8 zu den entsprechenden Diamino-Derivaten (Verbindungen 9 bis 14) umgesetzt. Diese Diamino-Derivate, die dünnschichtchromatographisch und IR-spektroskopisch charakterisiert wurden, wurden unmittelbar zur Herstellung der folgenden cis-Platin-Komplexe eingesetzt:

2,4-Di-amino-1,6-anhydro-2,4-di-desoxy-3-O-methyl-ß-D-glucopyranose (Verbindung 9)

2,4-Di-amino-1,6-anhydro-3-O-decyl-2,4-di-desoxy-ß-D-glucopyranose (Verbindung 10)

2,4-Di-amino-1,6-anhydro-3-O-benzyl-2,4-di-desoxy-ß-D-glucopyranose (Verbindung 11)

2,4-Di-amino-1,6-anhydro-2,4-di-desoxy-3-O-tetrahydropyranyl-ß-D-glucopyranose (Verbindung 12)

2,4-Di-amino-1,6-anhydro-2,4-di-desoxy-3-O-methansulfonyl-ß-D-glucopyranose (Verbindung 13)

3-O-Acetyl-2,4-di-amino 1,6-anhydro-2,4-di-desoxy-ß-D-glucopyranose (Verbindung 14)

Beispiel 3 (Herstellung von Platinkomplexen)

2N,4N-(2,4-Di-amino-1,6-anhydro-2,4-di-desoxy-ß-D-glucopyranose)-platin(II)-di-chlorid (Verbindung 15)

5 g (29 mMol) Verbindung 8 wurden in 30 ml destilliertem, entgastem Wasser gelöst. Unter Rühren wurde die Lösung innerhalb 2 Stunden zu 13,6 g Kaliumtetrachloroplatinat, gelöst in 30 ml Wasser zugetropft. Der Reaktionsansatz wurde noch 20 Stunden unter Lichtausschluß bei Raumtemperatur gerührt, wobei im Verlauf der Reaktion die Verbindung 15 als Niederschlag ausfiel. Das Dünnschichtchromatogramm der Reaktionslösung (Laufmittel: Chloroform/Methanol/Wasser 4:4:1, Sprühreagenzien: Ninhydrin und Zinndichlorid) zeigte, daß die Ninhydrin-aktive Ausgangsverbindung vollständig umgesetzt war. Der Niederschlag wurde bei 0°C abfiltriert, mit Wasser gewaschen und in DMF und Wasser umkristallisiert. Das Filtrat wurde i.Vak. auf ca. 5 ml Volumen eingeengt und auf 0°C abgekühlt. Die ausgefallene Verbindung 15 wurde in gleicher Weise wie die Hauptfraktion aufgearbeitet. Das Produkt wurde noch 3 Tage im Hochvakuum getrocknet.

Ausbeute: 11,8 g (93 %)
$^1H$-NMR (400 MHz, DMSO-$d_6$): delta 1-H 5,45s, 2-H 2,16d, 3-H 3,27s, 4-H 2,06d, 5-H 4,63d, 6-$H_A$ 4,14d, 6-$H_B$ 3,43dd, NH 4,72d, 4,87d, 5,51dd, 5,58dd ppm

berechnet (%):C 16,91 H 2,83 N 6,57 Pt 45,77 Cl 16,6
gefunden (%): C 16,83 H 2,81 N 6,42 Pt 45,56 Cl 16,7

Analog wurden folgende Verbindungen synthetisiert:

2N,4N-(2,4-Di-amino-1,6-anhydro-2,4-di-desoxy-3-O-methyl-ß-D-glucopyranose)-platin(II)-di-chlorid (Verbindung 16)

IR (KBr, $cm^{-1}$): 3500, 3245-3100, 2950, 1570, 1450

berechnet (%):C 19,09 H 3,20 N 6,36 Pt 44,32 Cl 16,1
gefunden (%): C 19,12 H 3,21 N 6,32 Pt 44,18 Cl 16,0

2N,4N-(2,4-Di-amino-1,6-anhydro-3-O-decyl-2,4-di-desoxy-ß-D-glucopyranose)-platin(II)-di-chlorid (Verbindung 17)

IR (KBr, $cm^{-1}$): 3500, 3245-3100, 2950-2850

berechnet (%): C 11,82 H 1,98 N 4,59 Pt 32,03 J 41,6
gefunden (%): C 11,73 H 1,96 N 4,39 Pt 32,97 J 41,3

2N,4N-(2,4-Di-amino-1,6-anhydro-3-O-benzyl-2,4-di-desoxy-ß-D-glucopyranose)-platin(II)di-chlorid (Verbindung 18)

IR (KBr, $cm^{-1}$): 3500,3250,3200,3100,2960,2900,1590,1500

berechnet (%):C 30,24 H 3,51 N 5,42 Pt 37,78 Cl 13,73
gefunden (%): C 30,12 H 3,51 N 5,37 Pt 36,9 Cl 13,51

2N,4N-(2,4-Di-amino-1,6-anhydro-2,4-di-desoxy-3-O-tetrahydropyranyl-ß-D-glucopyranose)-platin(II)-di-chlorid (Verbindung 19)

IR (KBr, $cm^{-1}$): 3500, 3250, 3200, 3100, 2950, 1590

berechnet (%): C 25,94 H 3,76 N 5,50 Pt 38,31 Cl 13,9
gefunden (%): C 26,01 H 3,78 N 5,43 Pt 38,02 Cl 14,1

2N,4N-(2,4-Di-amino-1,6-anhydro-2,4-di-desoxy-3-O-methan-sulfonyl-ß-D-glucopyranose)-platin(II)-di-chlorid (Verbindung 20)

IR (KBr, $cm^{-1}$): 3500, 3250, 3200, 2950, 1590

ber.(%): C 16,67 H 2,79 N 5,55 Pt 38,68 Cl 14,06 S 6,35
gef.(%): C 16,52 H 2,80 N 5,45 Pt 38,52 Cl 14,00 S 6,20

2N,4N-(3-O-Acetyl-2,4-di-amino-1,6-anhydro-2,4-di-desoxy-ß-D-glucopyranose)-platin(II)-di-chlorid (Verbindung 21)

IR (KBr, $cm^{-1}$): 3500, 3250-3200, 2950, 1700, 1610

berechnet (%): C 20,52 H 3,01 N 5,98 Pt 41,66 Cl 15,14
gefunden (%): C 20,53 H 3,02 N 5,83 Pt 40,94 Cl 15,02

2N,4N-(2,4-Di-amino-1,6-anhydro-2,4-di-desoxy-ß-D-glucopyranose)-platin(II)-dijodid (Verbindung 22)

1,0 g (2,44 mMol) Kaliumtetrachloroplatinat und 4,0 g (24,4 mMol) Kaliumjodid wurden in 15 ml Wasser suspendiert. Nach 30 min wurden in die entstandene Lösung unter Rühren 0,42 g (2,44 mMol) Verbindung 8, gelöst in 5 ml Wasser, innerhalb von 1 Stunde zugetropft. Das Reaktionsgemisch wurde 20 Stunden bei Raumtemperatur unter Lichtausschluß gerührt, wobei die Verbindung 22 als Niederschlag ausfiel. Der Niederschlag wurde bei 0°C abfiltriert, mit Wasser und Ethanol gewaschen und aus Wasser und DMF umkristallisiert.
Das Produkt wurde noch 3 Tage im Hochvakuum getrocknet.

Ausbeute: 1,28 g (86%)
$^1$H-NMR (400 MHz, DMSO-$d_6$): delta 1-H 5,45s, 2-H 2,18d, 3-H 3,28s, 4-H 2,03d, 5-H 4,63d, 6-$H_A$ 4,14d, 6-$H_B$ 3,43dd, NH 4,78d, 4,82d, 5,63dd, 5,59dd ppm

| | | | | | |
|---|---|---|---|---|---|
| berechnet (%): | C 11,82 | H 1,98 | N 4,59 | Pt 32,03 | J 41,6 |
| gefunden (%): | C 11,73 | H 1,96 | N 4,39 | Pt 31,97 | J 41,3 |

<u>Beispiel 4</u> Umsetzung von Verbindung 15 mit Silber(I)salzen von anorganischen und organischen Säuren

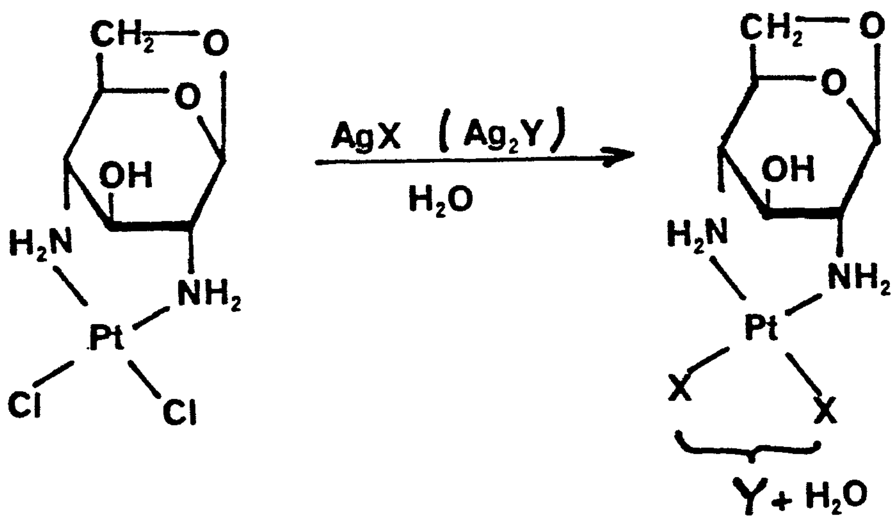


Hierbei bedeuten X ein einwertiges Anion wie Nitrat, Acetat, Trifluoracetat, Trifluorsulfonat, Toluolsulfonat, Perchlorat etc. und Y ein zweiwertiges Anion wie Sulfat oder Carbonat, wobei die Platinverbindung auch als Aquo-Komplex vorliegen kann.

Die IR-spektra dieser Verbindungen stimmen weitgehend überein mit den Banden der Diamino-Ligande und unterscheiden sich nur in den Banden, die für das betreffende Anion charakteristisch sind.

2N,4N-(2,4-Di-amino-1,6-anhydro-2,4-di-desoxy-ß-D-gluco-pyranose)-platin(II)-dinitrat (Verbindung 23)

10 g (23,47 mMol) Verbindung 15 wurden in 500 ml destilliertem Wasser gelöst. Nach Zugabe von 7,97 g Silbernitrat wurde der Reaktionsansatz 40 Stunden bei RT unter Lichtausschluß gerührt. Dann wurde das ausgefallene Silberchlorid abfiltriert und das Filtrat wurde i.Vak. unter Lichtausschluß auf 40 ml Volumen eingeengt. Die hierbei ausgefallene Verbindung 23 wurde abfiltriert und 2 Tage im Hochvakuum getrocknet.

Ausbeute: 10,68 g (95%)
IR (KBr, $cm^{-1}$): 3400, 3200, 3150, 1600, 1500, 1390, 1275

| | | | | |
|---|---|---|---|---|
| berechnet (%): | C 15,03 | H 2,52 | N 11,68 | Pt 40,70 |
| gefunden (%): | C 15,21 | H 2,54 | N 11,72 | Pt 39,92 |

Analog der Vorschrift zur Herstellung der Verbindung 23 wurden folgende Verbindungen synthetisiert:

2N,4N-(2,4-Di-amino-1,6-anhydro-2,4-di-desoxy-ß-D-gluco-pyranose)-platin(II)-diacetat (Verbindung 24)

| | | | | |
|---|---|---|---|---|
| berechnet (%): | C 25,37 | H 3,83 | N 5,91 | Pt 41,21 |
| gefunden (%): | C 25,42 | H 3,87 | N 5,63 | Pt 40,02 |

2N,4N-(2,4-Di-amino-1,6-anhydro-2,4-di-desoxy-ß-D-gluco-pyranose)-platin(II)-bis(trifluoracet (Verbindung 25)

| | | | | | |
|---|---|---|---|---|---|
| berechnet (%): | C 20,66 | H 2,08 | N 48,19 | Pt 33,56 | F 19,61 |
| gefunden (%): | C 20,73 | H 2,09 | N 47,83 | Pt 32,82 | F 19,21 |

2N,4N-(2,4-Di-amino-1,6-anhydro-2,4-di-desoxy-ß-D-gluco-pyranose)-platin(II)-bis(trifluormethansulfonat) (Verbindung 26)

ber.(%): C 14,70 H 1,85 N 4,28 Pt 29,85 S 9,81 F 17,4
gef.(%): C 14,72 H 1,91 N 4,26 Pt 29,47 S 9,73 F 17,2

2N,4N-(2,4-Di-amino-1,6-anhydro-2,4-di-desoxy-ß-D-gluco-pyranose)-platin(II)-bis(p-toluolsulfonat) (Verbindung 27)

berechnet (%): C 34,41 H 3,75 N 4,01 Pt 27,96 S 9,19
gefunden (%): C 34,52 H 3,69 N 3,91 Pt 27,55 S 9,03

2N,4N(2,4-Di-amino-1,6-anhydro-2,4-di-desoxy-ß-D-gluco-pyranose)-platin(II)-diperchlorat (Verbindung 28)

berechnet (%): C 13,00 H 2,18 N 5,05 Pt 35,20 Cl 12,8
gefunden (%): C 12,85 H 2,14 N 5,01 Pt 35,07 Cl 12,7

2N,4N-(2,4-Di-amino-1,6-anhydro-2,4-di-desoxy-ß-D-gluco-pyranose)-platin(II)-hydroxid (Verbindung 29)

5 g (10,43 mMol) Verbindung 23 wurden in 250 ml Wasser gelöst. Unter Rühren wurden 25 g Ionenaustausch-Harz (Dovex, Type 1 x 8; Aktivierung mit 10N NaOH) zugegeben. Nach 30 min zeigte das Dünnschichtchromatogramm (n-Butanol/Eisessig/Wasser 5:3:2, Cellulose-Folie), daß der Austausch des Nitrates durch Hydroxyl vollständig abgelaufen war. Nach Abfiltrieren des Harzes wurde das Filtrat zur Trocknung unter Lichtausschluß i.Vak. eingeengt.

Ausbeute: 3,7 g(91%) $(\alpha)^{20}_{258}$ = -23° (c = 1 $H_2O$)
IR (KBr, $cm^{-1}$): 3500-2900, 1600, 1200

berechnet (%): C 18,51 H 3,62 N 7,19 Pt 50,11
gefunden (%): C 18,23 H 3,65 N 7,01 Pt 49,63

Beispiel 5

Die Herstellung von weiteren cis-Platin-Komplexen erfolgte nach folgender Gleichung ausgehend von Verbindungen 23 und 29,

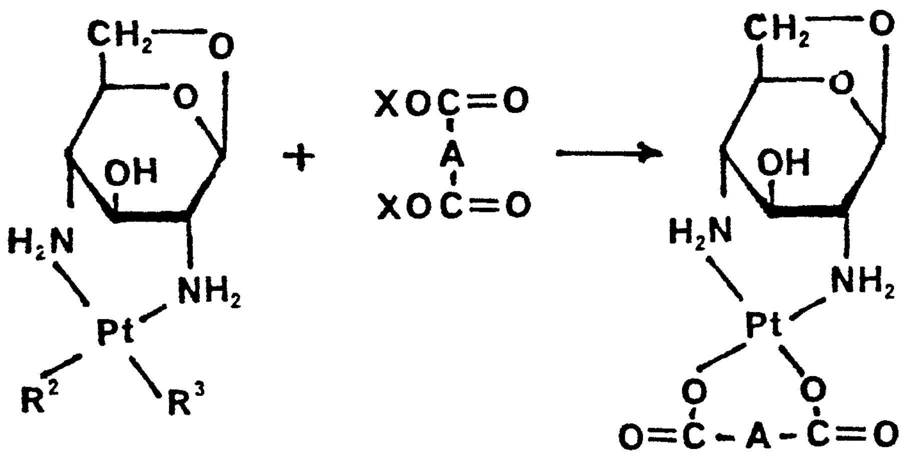

wobei $R^2$ und $R^3$ entweder $NO_3^-$ oder $OH^-$, X = H oder Alkalisalz wie Natrium, Kalium und OOC-A-COO Anion einer Di-, Oligo- oder Polycarbonsäure bedeuten.

a) Herstellung von cis-Platin-Komplexen ausgehend von Verbindung 23

2N,4N-(2,4-Di-amino-1,6-anhydro-2,4-di-desoxy-ß-D-glucopyranose)-platin(II)-malonat (Verbindung 30)

3,4 g (7,1 mMol) Verbindung 23 wurden in 50 ml $H_2O$ gelöst. Zu dieser Lösung wurden 1,05 g (7,1 mMol) Natriumalonat, gelöst in 10 ml Wasser, langsam zugetropft. Nach 16 Stunden Rühren unter Lichtausschluß zeigte das Dünnschichtchromatogramm (n-Butanol/Eisessig/Wasser 5:3:2), daß die Umsetzung abgeschlossen war. Die Reaktionslösung wurde auf ca. 5 ml eingeengt, wobei die Verbindung 30 ausgefallen ist. Der Niederschlag wurde noch mit Eiswasser gewaschen und anschließend 3 Tage im Hochvakuum getrocknet.

Ausbeute: 2 g (62%) $(\alpha)^{20}_{589}$ = -21° (c = 1 $H_2O$)
$^{13}C$-NMR (90 MHz, $D_2$):

| | | | | |
|---|---|---|---|---|
| berechnet (%): | C 23,63 | H 3,08 | N 6,12 | Pt 42,66 |
| gefunden (%): | C 23,72 | H 3,11 | N 6,10 | Pt 41,97 |

b) Herstellung von cis-Platin-Komplexen ausgehend von Verbindung 29

2N,4N-(2,4-Di-amino-1,6-anhydro-2,4-di-desoxy-ß-D-glucopyranose)-platin(II)-malonat (Verbindung 30)

5 g (12,8 mMol) Verbindung 29 wurden in 50 ml destilliertem Wasser gelöst. Zu dieser Lösung wurden 1,33 g (12,8 mMol) Malonsäure, gelöst in 5 ml Wasser, unter Rühren und Lichtausschluß zugetropft. Nach 6 Stunden wurde die Reaktionslösung i.Vak. eingedampft und anschließend im Hochvakuum getrocknet.

Ausbeute: 5,8 g (98%) $(\alpha)^{20}_{589}$ = -21° (c = 1 $H_2O$)

| | | | | |
|---|---|---|---|---|
| berechnet (%): | C 23,63 | H 3,08 | N 6,12 | Pt 42,66 |
| gefunden (%): | C 23,52 | H 3,12 | N 6,03 | Pt 41,92 |

Nach der Methode a) bzw. b) wurden folgende cis-Platin-Komplexe hergestellt. Diese Verbindungen wurden meist IR-spektroskopisch bzw. NMR-spektroskopisch charakterisiert.

2N,4N-(2,4-Di-amino-1,6-anhydro-2,4-di-desoxy-ß-D-glucopyranose)-platin(II)-oxalat (Verbindung 31)

| | | | | |
|---|---|---|---|---|
| berechnet (%): | C 21,67 | H 2,72 | N 6,32 | Pt 44,01 |
| gefunden (%): | C 21,91 | H 2,78 | N 6,21 | Pt 43,73 |

2N,4N-(2,4-Di-amino-1,6-anhydro-2,4-di-desoxy-ß-D-gluco-pyranose)-platin(II)-hydroxymalonat (Verbindung 32)

| | | | | |
|---|---|---|---|---|
| berechnet (%): | C 22,84 | H 2,98 | N 5,91 | Pt 41,21 |
| gefunden (%): | C 22,62 | H 2,97 | N 5,82 | Pt 40,73 |

2N,4N-(2,4-Di-amino-1,6-anhydro-2,4-di-desoxy-ß-D-gluco-pyranose)-platin(II)-cyclobutan-1,2-dicarboxylat (Verbindung 33)

| | | | | |
|---|---|---|---|---|
| berechnet (%): | C 28,97 | H 3,64 | N 5,63 | Pt 39,22 |
| gefunden (%): | C 28,91 | H 3,71 | N 5,42 | Pt 38,71 |

2N,4N-(2,4-Di-amino-1,6-anhydro-2,4-di-desoxy-ß-D-gluco-pyranose)-platin(II)-cyclobutan-1,1-dicarboxylat (Verbindung 34)

| | | | | |
|---|---|---|---|---|
| berechnet (%): | C 28,97 | H 3,64 | N 5,63 | Pt 39,22 |
| gefunden (%): | C 28,73 | H 3,61 | N 5,43 | Pt 39,02 |

2N,4N-(2,4-Di-amino-1,6-anhydro-2,4-di-desoxy-ß-D-gluco-pyranose)-platin(II)-2,5,8-trioxanonan-1,9-dicarboxylat (Verbindung 35)

| | | | | |
|---|---|---|---|---|
| berechnet (%): | C 29,22 | H 4,20 | N 4,86 | Pt 33,90 |
| gefunden (%): | C 29,31 | H 4,20 | N 4,73 | Pt 33,32 |

2N,4N-(2,4-Di-amino-1,6-anhydro-2,4-di-desoxy-ß-D-gluco-pyranose)-platin(II)-3-carboxyphthalat (Verbindung 36)

| | | | | |
|---|---|---|---|---|
| berechnet (%): | C 31,98 | H 2,86 | N 4,97 | Pt 31,95 |
| gefunden (%): | C 31,83 | H 2,81 | N 4,83 | Pt 31,27 |

2N,4N-(2,4-Di-amino-1,6-anhydro-2,4-di-desoxy-ß-D-gluco-pyranose)-platin(II)-4-carboxyphthalat (Verbindung 37)

| | | | | |
|---|---|---|---|---|
| berechnet (%): | C 31,98 | H 2,86 | N 4,97 | Pt 31,95 |
| gefunden (%): | C 31,73 | H 2,85 | N 4,79 | Pt 30,96 |

2N,4N-(2,4-Di-amino-1,6-anhydro-2,4-di-desoxy-ß-D-glucopyranose)-platin(II)-maleinat (Verbindung 38)

| | | | | |
|---|---|---|---|---|
| berechnet (%): | C 25,59 | H 3,00 | N 5,96 | Pt 41,56 |
| gefunden (%): | C 25,67 | H 3,02 | N 5,82 | Pt 40,73 |

2N,4N-(2,4-Di-amino-1,6-anhydro-2,4-di-desoxy-ß-D-glucopyranose)-platin(II)-aconit (Verbindung 39)

| | | | | |
|---|---|---|---|---|
| berechnet (%): | C 27,38 | H 2,87 | N 5,32 | Pt 37,06 |
| gefunden (%): | C 27,32 | H 2,84 | N 5,10 | Pt 36,38 |

2N,4N-(2,4-Di-amino-1,6-anhydro-2,4-di-desoxy-ß-D-glucopyranose)-platin(II)-Komplex mit Polyglutaminsäure (Verbindung 36)

80,4 mg Poly-D-glutaminsäure (MG 4000) wurden in 2 ml Wasser gelöst und mit 0,5 N NaOH auf pH 6,5 eingestellt. Diese Lösung wurde zu 150 mg (0,313 mMol) Verbindung 23, gelöst in 15 ml Wasser, zugegeben. Nach 18 Stunden Rühren wurde die entstandene Suspension of 0°C abgekühlt und abfiltriert. Der abfiltrierte Niederschlag wurde noch mit Eiswasser gewaschen und in Hochvakuum getrocknet.

Ausbeute: 150 mg
berechnet (%): Pt 27,3
gefunden (%): Pt 26,8

Patentansprüche:

1. Verbindung der allgemeinen Formel I

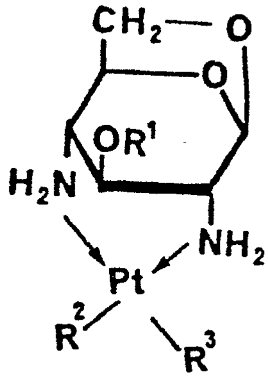

I

worin

$R^1$ ein Wasserstoffatom, eine Alkylgruppe mit 1 bis 16 Kohlenstoffatomen oder eine in der Kohlenhydratchemie übliche Schutzgruppe, besonders eine Benzyl-, Tetrahydropyranyl-, Acetyl-, Halogenacetyl-, Benzoyl-, Methansulfonyl- oder para-Toluolsulfonyl-Gruppe,
$R^2$ und $R^3$ gleich sind und Chlorid, Bromid oder Jodid oder Hydroxy, Nitrat, Acetat, Trifluoracetat, Trifluormethansulfonat, Toluolsulfonat oder Perchlorat oder
$R^2$ Sulfat oder Carbonat und $R^3$ $H_2O$ oder
$R^2$ und $R^3$ zusammen das Dianion einer organischen Säure, besonders der Oxal-, Malon-, Hydroxymalon-, Bernstein-, Malein-, Aconit-, 3,6,9-Trioxaundecandi-, 1,1- oder 1,2-Cyclobutandicarbon-, 3- oder 4-Carboxyphthal- oder 3,4-Dicarboxyphthalsäure oder
$R^2$ und $R^3$ zusammen eine wiederkehrende anionische Einheit einer polymeren Verbindung, besonders Dextran-, Chondroitin- oder Galactansulfat, Polyglutaminsäure oder Polyitaconsäure darstellen.

2. Verbindung der allgemeinen Formel I, worin
$R^1$ ein Wasserstoffatom, eine Alkylgruppe mit 1-10 Kohlenstoffatomen oder eine Benzyl-, Tetrahydropyranyl-, Acetyl- oder Methansulfonylgruppe, $R^2$ und $R^3$ gleich sind und Chlorid, Jodid, Nitrat, Hydroxy, Acetat, Trifluoracetat, Trifluormethansulfonat, Toluolsulfonat oder Perchlorat oder $R^2$ Sulfat oder Carbonat und $R^3$ $H_2O$ oder $R^2$ und $R^2$ zusammen das Dianion von Oxal-, Malon-, Hydroxymalon-, Bernstein-, Malein, Aconit-, 3,6,9-Trioxaundecandi-, 1,1- oder 1,2-Cyclobutandicarbon-, 3- oder 4-Carboxyphthal-oder 3,4-Dicarboxyphthalsäure oder $R^2$ und $R^3$ zusammen eine wiederkehrende anionische Einheit einer polymeren Verbindung, besonders Dextransulfat, Chondroitinsulfat, Galactansulfat, Polyglutaminsäure oder Polyitaconsäure darstellen.

3. Verfahren zur Herstellung einer Verbindung nach Anspruch 1, dadurch gekennzeichnet, daß man

a) eine Verbindung der allgemeinen Formel II

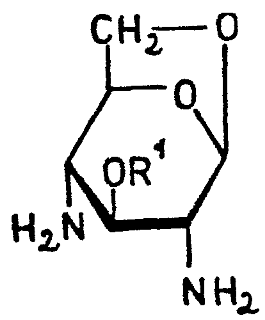

II

in der $R^1$ ein Wasserstoffatom oder eine Alkylgruppe mit 1 bis 16 Kohlenstoffatomen oder eine in der Kohlenhydratchemie übliche Schutzgruppe, besonders eine Benzyl-, Tetrahydropyranyl-, Acetyl-, Halogenacetyl-, Benzoyl-, Methansulfonyl- oder para-Toluolsulfonyl-Gruppe ist, mit $K_2PtX_4$, worin X ein Chlorid- oder Jodid-Ion darstellt, in Gegenwart eines Lösungsmittels wie Wasser, DMF oder DMSO oder deren Mischungen mit THF, Dioxan, Methanol oder Ethylenglycoldimethylether bei einer Temperatur von 0°C bis 80°C,

bevorzugt 10°C bis 40°C, in an sich bekannter Weise zu einem Reaktionsprodukt der allgemeinen Formel I umsetzt, in der $R^1$ die oben angegebene Bedeutung besitzt und $R^2$ und $R^3$ Chlorid oder Jodid bedeuten, und diese Verbindung gegebenenfalls in eine weitere Verbindung nach Anspruch 1 umsetzt.

4. Verwendung einer Verbindung nach Anpruch 1 in einem Arzneimittel.